# EUROPEAN PATENT SPECIFICATION

(11) **EP 0 439 932 B1**
(45) Date of publication and mention of the grant of the patent: **02.11.1994**
(21) Application number: 90313392.4
(22) Date of filing: 10.12.1990
(51) Int. Cl.: A61M 25/01, A61B 17/22

(54) **Telescoping guide catheter system**
Teleskopisches Führungskathetersystem
Système télescopique de cathéter à guidage

(30) Priority: 12.01.1990 US 464324
(43) Date of publication of application: 07.08.1991
(73) Proprietor: SCHNEIDER (USA) INC., a Pfizer Company, Plymouth,Minnesota (US)
(72) Inventor: Shockey, Rick L., Eagan, Minnesota (US); Baim, Donald S., Newton, Massachusetts (US); Cronk, Kevin L., Maple Grove, Minnesota (US); Campbell, Rocky Roy James, Maple Plain, Minnesota (US)
(74) Representative: Hayles, James Richard

(56) References cited:
- EP-A- 0 277 366
- EP-A- 0 303 487
- US-A- 4 323 071
- US-A- 4 531 943
- US-A- 4 581 017
- US-A- 4 636 346
- US-A- 4 774 949

## Description

This invention relates generally to improved catheter apparatus for facilitating the recanalization of a stenosed coronary artery and more particularly to an improved guide catheter system whereby a guide catheter member can be advanced beyond the coronary ostium and into the coronary artery itself up to the point of the stenosis.

In treating coronary artery disease, a variety of surgical techniques are employed to recanalize an occluded or partially occluded artery segment without requiring open heart surgery. Using the technique pioneered by A. Gruntzig, a catheter having an expander (balloon) at its distal end is routed through the vascular system and ultimately into the coronary artery with the balloon being juxtaposed with the stenotic lesion. Once so positioned, the balloon is inflated to compress the plaque into the wall of the blood vessel, thus restoring patency.

In another procedure, referred to as an atherectomy, a catheter having a rotatable cutter at its distal tip is advanced through the vascular system and when the tip is made to abut the atheroma, a motor at the proximal end of the catheter is used to drive the cutter to surgically "tunnel" through the lesion. In this regard, reference is made to the Rydell U.S. Patent 4,784,636, assigned to applicant's assignee.

Still others have incorporated a fiber optic bundle in a catheter and a laser is used to burn through the plaque comprising the arterial blockage. In this regard, reference is made to the Moore et al U.S. Patent 4,669,465, assigned to GV Medical, Inc., of Minneapolis, Minnesota.

The foregoing are exemplary of working catheters, i.e., the catheters that are directly involved in the recanalization through expansion, excision or ablation. To properly position with working catheter, it is also necessary that a guide catheter be utilized. A guide catheter of the prior art typically comprises an elongated, flexible tube having an internal lumen sufficiently large to receive and pass the working catheter therethrough. In that the catheters are generally introduced into the femoral artery and then advanced through the vascular system to the heart, the guide catheter must possess a characteristic of "torqueability" meaning that it can transmit a twisting force applied at its proximal end to the distal end to facilitate the ability to steer it through the appropriate vascular branches. The torqueability characteristic is achieved by the appropriate choice of materials for the guide catheter shaft or by incorporating a braid sheath of wire strands embedded in the wall of the guide catheter. Those wishing further information concerning the construction of a typical prior art catheter are referred to the Stevens U.S. Patent 3,485,234, assigned to the Cordis Corporation of Miami, Florida.

EP-A-277366, which forms the closest prior art with regard to the present invention, describes a catheter assembly in which a second guiding catheter is arranged within the bore of a first guiding catheter.

When it is considered that the lumen of the guide catheter must be large enough to pass the working catheter and that the guide catheter shaft must exhibit acceptable torqueability and stiffness characteristics, it tends to dictate a guide catheter having a relatively large outside diameter. In fact, it has not been possible in the past to advance the distal end of the conventional guide catheter beyond the coronary ostium. Where the site of the lesion in the coronary artery is several centimeters beyond the ostium and it becomes necessary during the recanalization procedure to exchange working catheters, damage may be done to the delicate tissue of the intima.

The invention provides an angioplasty guide catheter system comprising an outer guide catheter comprising an elongated flexible plastics tube having an outside diameter small enough to pass through the vascular system from an introducer site to the coronary ostia, and an internal lumen of a size to pass an inner guide catheter, the outer catheter including a reinforcing means internal to the tube wall, and an inner guide catheter comprising an elongated flexible tubular core of a lubricious material surrounded by a coating of polymeric material without reinforcing means small enough to fit within the lumen of the outer guide catheter, the lumen of the tubular core being large enough to pass a working catheter therethrough. The second guide catheter may comprise an elongated thin-wall tube of polytetrafluoroethylene (Teflon®) which is coated on its exterior with a blend of polyurethanes. Because the second guide catheter is to be passed through the lumen of the first guide catheter, it does not require a braided layer to provide the torqueability necessary to route the guide catheter from its femoral artery entry point to the heart. Instead, it tracks the lumen of the first guide catheter. Because the need for a braided layer is obviated, it can be made of a sufficiently thin wall that its outside diameter is sufficiently small that the second guide catheter can be advanced beyond the ostium and into the coronary artery itself. To minimize damage to the endothelial layer, the second guide catheter is equipped with a soft tip which is appropriately rounded to avoid sharp edges which can damage the arterial wall. The soft tip may preferably be formed from a polyurethane resin exhibiting low durometer as compared to the durometer of the polyurethane blend coating the Teflon tubular core. By lining the second guide catheter with the Teflon core, its trackability and pushability property is enhanced. The Teflon layer provides a low coefficient of friction with the working catheter to be inserted.

With the second guide catheter advanced so that its distal end portion is contained within the coronary artery to be treated, it provides backup support for the blood vessel during the recanalization procedure and allows repeated exchanges of working catheters with a minimum of damage to the intima.

The foregoing features, objects and advantages of the invention will become more apparent to those skilled in the art from the following detailed description of a preferred embodiment, especially when considered in conjunction with the accompanying drawings in which like numerals in the several views refer to corresponding parts.
Figure 1 is a side view, partially cross-sectioned of the guide catheter system of the present invention; and
Figure 2 is a diagram illustrating the use of the present invention in recanalizing a stenosed coronary arterial blood vessel.

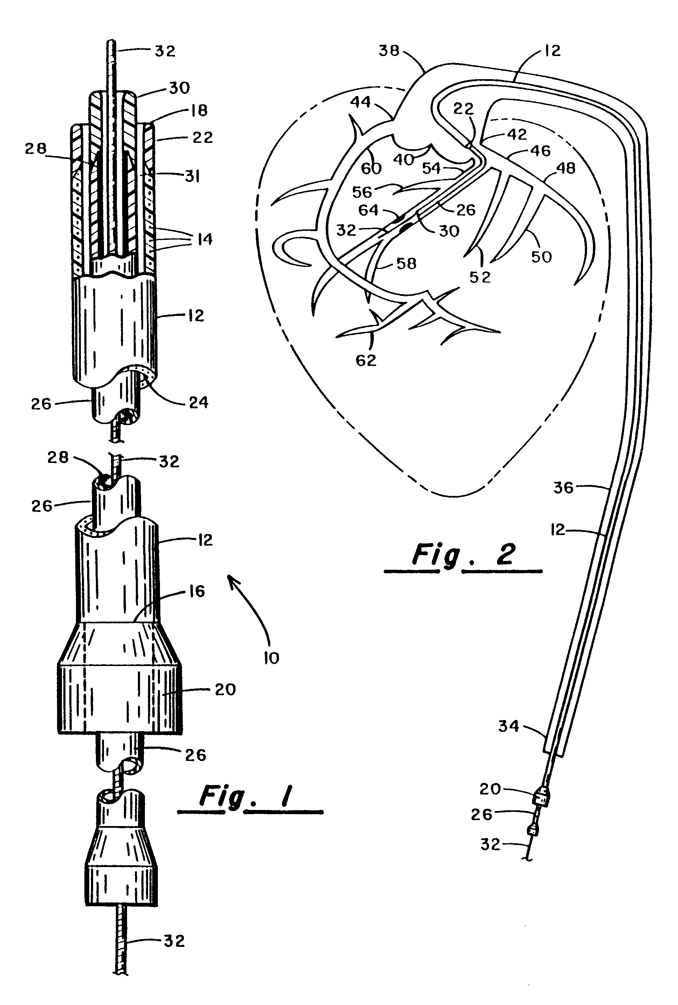

Referring first to Figure 1, there is indicated generally by numeral 10 the guide catheter system in accordance with the present invention. It is seen to include a first, relatively large diameter guide catheter 12, comprising an elongated, flexible plastic tube which would typically approximately 100 cms. in length and fabricated primarily from a blend of polyurethanes and containing a braided wire sheath 14 within its walls. In that the outer guide catheter 12 is intended to be routed through the vascular system from a peripheral site such as an incision into the femoral artery, the wire braided affords the desirable torque properties whereby twisting the proximal end 16 will transmit a corresponding torque to the distal end 18 of the catheter 12. The catheter 12 may be in the range of from about 3 mm to 1.7 mm (9 French to 5 French) and designed to have an internal lumen in the range of from 2,3 to 1,1 mm (7 French to 3.5 French). Bonded to the proximal end 16 of the outer catheter 12 is a conventional catheter hub 20. The catheter structure thus far described may be produced in accordance with the Stevens U.S. Patent 3,485,234 which describes a manufacturing process whereby a catheter is produced having a precisely dimensioned lumen and a braided wire sheath disposed entirely within the plastic comprising the catheter's wall.

To reduce the likelihood and/or extent of trauma to the endothelial lining of she blood vessels through which the outer catheter 12 is routed, it is preferably provided with an atraumatic tip 22 which is appropriately attached to the end of the outer tubing member. The soft tip 22 will typically be formed from a polyurethane blend exhibiting a lower durometer than the plastic comprising the remainder of the catheter. It may be attached and otherwise configured as set out in the Van Tassel et al U.S. Patent 4,531,943 assigned to applicant's assignee.

Coaxially disposed and loosely fitting within the lumen 24 of the first or outer catheter 12 is a second catheter 26 which is of a somewhat greater length than the first catheter 12. The body of the catheter 26 comprises an internal tubular core 28 which is made from polytetrafluoroethylene (Teflon®) having a wall thickness of approximately 0,0125 mm (0.0005 inches). The Teflon tube, while supported on a cylindrical mandrel is then coated with a suitable plastic, preferably a blend of polyurethanes so that the composite outside diameter is in the range of from 2,6 to 1,0 mm (8 French to 3 French).

As mentioned above, the inside diameter of the outer guide catheter 12, if designed to be 2,3 mm (7 French,) leaves a predetermined clearance between the two when a second catheter 26 of, say, 2 mm (6 French), is telescopingly received within the lumen 24 of the outer catheter 12. This permits the perfusion or aspiration of liquids through the annular gap between inner wall of the outer catheter 12 and the outer wall of the inner catheter 26.

Appropriately bonded to the distal end portion of the second or inner catheter 26 is an atraumatic tip 30 again formed from a suitable plastic material existing low durometer.

The catheter 26, being 2 mm (6 French), may have an internal diameter of 1,5 mm (0.063 inches) which is sufficiently large to pass a working catheter (not shown) therethrough. As mentioned above, the working catheter employed may include angiography, angioplasty, atherectomy as well as various pressure monitoring or fiber optic devices.

Shown threaded through the lumen of the inner tubular catheter 26 is an elongated flexible, helically wound stainless steel guidewire 32.

Having described the constructional features of the guide catheter system of the present invention, consideration will next be given to its mode of use. In this regard, reference is made to Figure 2 which diagrammatically depicts the various coronary arterial blood vessels, both anterior and posterior with the remainder of the heart eliminated. Thus, numeral 34 identifies the femoral artery leading to the descending aorta 36, the aortic arch 38 and the aortic valve 40. Located adjacent the aortic arch 38 and slightly above the valve 40 is the left coronary ostium 42 and the right coronary ostium 44.

Leading away from the ostium 42 is the left coronary artery 46 which joins to the circumflex branch 48 from which the obtuse marginal branch 50 and the intermediate branch 52 extend.

The left anterior descending artery is identified by numeral 54 and branching from it are the septal arteries 56 and 58.

Extending from the right coronary ostium 44 is the right coronary artery 60 which leads to the posterior descending artery 62. Let it be assumed that a stenosis exists at 64 in the left anterior descending artery 54 and that it is desired to treat the lesion with an angioplasty balloon type working catheter. In carrying out the procedure, an incision is made and an introducer (not shown) is inserted into the femoral artery 34. The first catheter 12 is then passed retrograde up the descending aorta 36 and beyond the aortic arch 38 until its distal tip 22 abuts the coronary ostium 42. Next, a guidewire 32 is passed through the lumen of the first guide catheter 12 and maneuvered by twisting and advancing distally until the guidewire passes down the left anterior descending artery 54 and beyond the location of the lesion 64. Once the guidewire 32 is so positioned, the telescoping guide catheter 26 is passed over the wire 32 by feeding the distal tip 30 over the guidewire and feeding it through the hub 20 of the outer guide catheter 12 and thence through the lumen 24 thereof until it exits the distal end 18 of the outer catheter through the left coronary ostium 42 and down the left anterior descending branch 54. Once the distal tip 30 of the, inner telescoping guide catheter 26 is proximate the site of the lesion, the working catheter (not shown) with its distal balloon may now be fed through the lumen 31 of the inner guide catheter. Because the interior wall of the inner guide catheter is lined with a layer of a lubricious plastic, such as Teflon, the coefficient of friction between the working catheter and the wall of the inner guide catheter is low, thus enhancing the trackability of the working catheter through the lumen of the inner guide catheter 26. Once the distal balloon of the working catheter is advanced across the stenosis, dilatation of the affected blood vessel can take place. Once the balloon has been advanced across the lesion, it is the physician's choice whether to pull the inner guide back into the lumen of the outer guide or not.

It may also occur that the physician will want to withdraw one working catheter in favor of a replacement working catheter. By leaving the internal guide catheter 26 in position, the exchange may take place without repeatedly drawing the working catheter back and forth over the endothelial lining of the blood vessel. Instead, the arterial branch remains supported by the guide catheter 26 during the exchanges.

## Claims

1. A guide catheter system (10) for use in performing coronary artery transluminal angioplasty or atherectomy procedures comprising:
(a) an outer guide catheter (12) comprising an elongated, flexible plastics tube having an outside diameter small enough to pass through the vascular system from an introducer site to the coronary ostia, and an internal lumen of a size to pass an inner guide catheter (26), said outer guide catheter (12) including a reinforcing means (14) internal to the wall of said tube; and
(b) said inner guide catheter (26) having a proximal end, a distal end and an outside diameter sufficiently small to fit with a predetermined clearance within said lumen of said outer guide catheter (12) and to pass into the coronary artery to be recanalized, the lumen of said tubular core being sufficiently large to pass a working catheter therethrough, characterized in that the inner guide catheter comprises an elongated flexible tubular core (28) of a lubricious material surrounded by a coating of polymeric material without reinforcing means.

2. The guide catheter system (10) as in Claim 1 and further including a tubular tip member (30) of a plastic material of relatively low durometer affixed to said distal end of said inner guide catheter (26), said tip member (30) having a rounded distal end for reducing damage to the endothelial lining of said coronary artery.

3. The guide catheter system as in Claim 1 wherein said outside diameter of said inner guide catheter (26) is in the range of from 2.7 to 1mm (8 French to 3 French) and the inside diameter of said outer guide catheter (12) is in the range of from 2.3 to 1.1mm (7 French to 3.5 French).

4. The guide catheter (10) as in Claim 1 and further including a molded plastic hub affixed to said proximal end of inner guide catheter (26).

## Patentansprüche

1. Führungskathetersystem (10) zur Verwendung bei der Durchführung transluminaler Angioplastie- oder Atherektomieverfahren in Koronararterien, umfassend:
(a) einen äußeren Führungskatheter (12), umfassend ein langgestrecktes, flexibles Kunststoffrohr mit einem Außendurchmesser, der klein genug ist, um ihn vom Ort einer Einführvorrichtung aus bis zum Koronarostium durch das Gefäßsystem hindurchzuführen, und einem inneres Lumen von einer Größe, um einen inneren Führungskatheter (26) hindurchzuführen, wobei der besagte äußere Führungskatheter (12) eine Verstärkungseinrichtung (14) im Inneren der Wand des besagten Rohrs einschließt; und
(b) wobei der besagte innere Führungskatheter (26) ein proximales Ende, ein distales Ende und einen Außendurchmesser aufweist, der ausreichend klein ist, um mit einem vorbestimmten Zwischenraum ins Innere des besagten Lumens des besagten äußeren Führungskatheters (12) zu passen und in die zu rekanalisierende Koronararterie einzutreten, wobei das Lumen des besagten röhrenförmigen Kerns ausreichend weit ist, um einen Arbeitskatheter hindurchzuführen, dadurch gekennzeichnet, daß der innere Führungskatheter einen langgestreckten, flexiblen, röhrenförmigen Kern (28) aus einem schlüpfrigen Material umfaßt, der von einem Überzug aus Polymermaterial ohne Verstärkungseinrichtungen umgeben ist.

2. Führungskathetersystem (10) nach Anspruch 1 und weiter enthaltend ein röhrenförmiges Spitzenelement (30) aus einem Kunststoffmaterial von relativ geringer Durometer-Härte, das an dem besagten distalen Ende des besagten inneren Führungskatheters (26) befestigt ist, wobei das besagte Spitzenelement (30) ein gerundetes distales Ende aufweist, um eine Schädigung des Endothelüberzugs der besagten Koronararterie zu vermindern.

3. Führungskathetersystem (10) nach Anspruch 1, bei welchem der besagte Außendurchmesser des besagten inneren Führungskatheters (26) im Bereich von 2,7 bis 1 mm (French-Größe 8 bis French-Größe 3) liegt, und der Innendurchmesser des besagten äußeren Führungskatheters (12) im Bereich von 2,3 bis 1,1 mm (French-Größe 7 bis French-Größe 3,5) liegt.

4. Führungskatheter (10) nach Anspruch 1 und weiter enthaltend ein an dem besagten proximalen Ende des inneren Führungskatheters (26) befestigtes geformtes Kunststoff-Ansatzstück.

## Revendications

1. Système (10) de cathéters de guidage à utiliser dans l'exécution d'opérations d'athérectomie ou d'angioplastie transluminale d'une artère coronaire, comportant :
(a) un cathéter extérieur (12) de guidage comportant un tube allongé et flexible en matière plastique ayant un diamètre extérieur assez petit pour passer dans le système vasculaire depuis un site d'introduction jusqu'à l'orifice coronaire, et une lumière intérieure d'une dimension permettant le passage d'un cathéter intérieur (26) de guidage, ledit cathéter extérieur (12) de guidage comprenant un moyen de renfort (14) à l'intérieur de la paroi dudit tube ; et
(b) ledit cathéter intérieur (26) de guidage ayant une extrémité proximale, une extrémité distale et un diamètre extérieur suffisamment petit pour s'ajuster avec un jeu prédéterminé à l'intérieur de ladite lumière dudit cathéter extérieur (12) de guidage et pour pénétrer dans l'artère coronaire devant être recanalisée, la lumière de ladite âme tubulaire étant suffisamment grande pour qu'un cathéter de travail puisse y passer, caractérisé en ce que le cathéter intérieur de guidage comporte une âme tubulaire flexible et allongée (28) en matière autolubrifiante entourée par un revêtement de matière polymérique sans moyens de renfort.

2. Système (10) de cathéters de guidage selon la revendication 1 et comprenant en outre un élément d'embout tubulaire (30) en matière plastique de dureté relativement faible, fixé à ladite extrémité distale dudit cathéter intérieur (26) de guidage, ledit élément d'embout (30) ayant une extrémité distale arrondie pour réduire la détérioration de la tunique endothéliale de ladite artère coronaire.

3. Système de cathéters de guidage selon la revendication 1, dans lequel ledit diamètre extérieur dudit cathéter intérieur (26) de guidage est dans la plage de 2,7 à 1 mm (8 French à 3 French) et le diamètre intérieur dudit cathéter extérieur (12) de guidage est dans la plage de 2,3 à 1,1 mm (7 French à 3,5 French).

4. Cathéter (10) de guidage selon la revendication 1, comprenant en outre un moyeu moulé en matière plastique fixé à ladite extrémité proximale du cathéter intérieur (26) de guidage.
